# FASCICULE DE BREVET EUROPEEN

(11) **EP 0 562 078 B1**
(45) Date de publication et mention de la délivrance du brevet: **04.10.1995**
(21) Numéro de dépôt: 92920662.1
(22) Date de dépôt: 11.09.1992
(51) Int. Cl.: A61K 7/09

(54) **PROCEDE DE DEFORMATION PERMANENTE DES CHEVEUX NE GENERANT PAS D'ODEUR DESAGREABLE**
VERFAHREN ZUR DAUERHAFTENVERFORMUNG VON HAAREN OHNE UNANGENEHMEN GERUCH ZU ERZEUGEN
NON-MALODOROUS HAIR PERMING METHOD

(30) Priorité: 13.09.1991 FR 9111325
(43) Date de publication de la demande: 29.09.1993
(73) Titulaire: L'OREAL, 75008 Paris (FR)
(72) Inventeur: SAMAIN, Henri, F-91570 Bièvres (FR); DUBIEF, Claude, F-78150 Le Chesnay (FR)
(74) Mandataire: Stalla-Bourdillon, Bernard
(86) Numéro de dépôt international: FR9200857
(87) Numéro de publication internationale: WO9305758

(56) Documents cités:
- WO-A-88/01860
- PATENT ABSTRACTS OF JAPAN vol. 8, no. 52 (C-213)(1489) 9 Mars 1984
- SEIFEN,OLE,FETTE,WACHSE vol. 117, no. 3, 21 Février 1991, AUGSBURG,DE pages 81 - 87 HOLLENBERG ET AL 'Moderne Stylingmittel-Funktion und Eigenschaften von Wellmitteln'
- DATABASE WPIL Section Ch, Week 8244, Derwent Publications Ltd., London, GB; Class D, AN 82-93495E

## Description

La présente invention a pour objet un nouveau procédé de déformation permanente des cheveux utilisant en tant qu'agent réducteur principal de la cystéamine, ce procédé comportant un traitement particulier permettant de combattre l'odeur désagréable résiduelle imprégnant les cheveux.

La technique classique pour réaliser la déformation permanente des cheveux consiste à réaliser, dans un premier temps, l'ouverture des ponts disulfures de la kératine à l'aide d'une composition contenant un agent réducteur (étape de réduction), puis, après avoir de préférence rincé la chevelure, à reconstituer dans un second temps lesdites liaisons disulfures en appliquant, sur les cheveux sous tension, une composition oxydante (étape d 'oxydation dite aussi de fixation) de façon à donner aux cheveux la forme définitive recherchée. L'application de la composition réductrice peut être effectuée avant ou après l'enroulement des cheveux sur les bigoudis.

Cette technique de déformation permanente des cheveux permet indifféremment de réaliser soit une ondulation des cheveux soit un défrisage ou décrêpage.

Les compositions pour réaliser le premier temps d'une opération de permanente se présentent généralement sous forme de lotions, de crèmes, de gels ou de poudres à diluer dans un support liquide, et contiennent, en tant qu'agent réducteur, de préférence un thiol.

Parmi les thiols préconisés pour réaliser le premier temps d'une opération de permanente, la cystéamine ou amino-2 éthanethiol s'est révélée particulièrement favorable pour l'obtention de bonnes frisures des cheveux mais l'on a constaté que les cheveux ainsi traités développaient parfois, après une semaine à un mois, une odeur très désagréable.

Cette mauvaise odeur est plus particulièrement perceptible lorsque les cheveux traités sont à l'état mouillé ou dans un environnement humide. Ainsi, l'apparition de mauvaises odeurs est tout particulièrement favorisée par un climat chaud et humide ou encore par une tendance du sujet à sécréter beaucoup de sébum.

Du fait de cet inconvénient, la cystéamine ainsi d'ailleurs que ses sels, même à de faibles concentrations, n'est généralement pas utilisée en tant qu'agent réducteur dans un procédé de déformation permanente des cheveux.

Après diverses recherches, on a constaté de façon surprenante et inattendue qu'il était possible de prévenir l'apparition des mauvaises odeurs dues à l'emploi de la cystéamine ou de l'un de ses sels, en procédant avant ou après l'étape de fixation, à l'application d'une composition contenant dans un véhicule cosmétiquement acceptable un composé mono aldéhydique.

La présente invention a donc pour objet un procédé de déformation permanente des cheveux comprenant une étape de réduction à l'aide d'une composition réductrice contenant de la cystéamine ou de l'un de ses sels et une étape d'oxydation, ce procédé étant caractérisé par le fait qu'avant ou après l'étape d'oxydation, on applique sur les cheveux une composition contenant dans un véhicule cosmétiquement acceptable un composé mono-aldéhydique pour combattre l'odeur désagréable résiduelle imprégnant les cheveux.

Comme composé mono-aldéhydique, on peut utiliser selon l'invention, le formaldéhyde ou un aldéhyde aromatique choisi parmi le 2,4,6-trihydroxybenzaldéhyde, l'α-méthyl β-(p-tert-butyl phényl) propionaldéhyde, le 2-méthyl-4-(2,6,6-triméthyl-2(1) cyclohéxene-1-yl) butanal, le 2-hexyl-3-phényl 2-propénal, le 3,4-diméthoxybenzaldéhyde, le 2,3,4-triméthoxybenzaldéhyde, le 2,5-diméthoxybenzaldéhyde et le 4-hydroxy 3-méthoxybenzaldéhyde.

De préférence, le composé mono-aldéhydique est utilisé à une concentration molaire comprise entre 0,15 et 1,8 mole et de préférence entre 0,22 et 0,8 mole.

Le véhicule cosmétiquement acceptable est une solution aqueuse ou une solution alcoolique ou hydroalcoolique d'un alcool aliphatique inférieur tel que l'éthanol ou l'isopropanol.

Selon une forme de réalisation préférée du procédé selon l'invention, les cheveux sont rincés à l'eau préalablement à l'application de la composition contenant le composé mono-aldéhydique.

La composition contenant le composé mono-aldéhydique peut bien entendu contenir divers ingrédients cosmétiques tels que par exemple un agent tensioactif anionique, nonionique ou amphotère ou zwitterionique ou cationique, un agent alcalinisant ou acidifiant, un agent conservateur, un stabilisant, un agent de traitement tel que des cations et des polymères, un colorant, un filtre solaire, un agent épaississant ou un agent nacrant.

Selon le procédé de l'invention, la composition contenant le composé mono-aldéhydique est appliquée avant ou après l'application de la composition oxydante pendant 1 à 60 minutes sur les cheveux. Après traitement, les cheveux peuvent être éventuellement rincés à l'eau sauf lorsque le composé mono-aldéhydique est le formaldéhyde auquel cas ils sont systématiquement rincés.

Selon une forme de réalisation particulièrement préférée du procédé selon l'invention, la composition contenant le composé mono-aldéhydique est appliquée sur les cheveux avant l'étape d'oxydation, les cheveux ayant été préalablement rincés à l'eau après l'étape de réduction.

Lorsque selon l'invention la composition contenant le composé mono-aldéhydique est appliquée après l'étape d'oxydation, l'application peut être immédiate ou différée dans le temps et éventuellement répétée une ou plusieurs fois.

Selon l'invention, la cystéamine ou l'un de ses sels est présente dans la composition réductrice à une concentration comprise entre 2 et 12% en poids en cystéamine base par rapport au poids total de la composition réductrice. La composition réductrice peut éventuellement contenir un agent réducteur secondaire tel que par exemple l'acide thioglycolique, le thioglycolate de glycérol ou la cystéine.

De préférence, le pH de la composition réductrice est compris entre 6 et 10 et est obtenu à l'aide d'un agent alcalin tel que par exemple l'ammoniaque, la monoéthanolamine, la diéthanolamine, la triéthanolamine, un carbonate ou bicarbonate alcalin ou d'ammonium, un hydroxyde alcalin ou à l'aide d'un agent acidifiant tel que par exemple l'acide chlorhydrique, l'acide acétique, l'acide lactique, l'acide oxalique, l'acide borique, l'acide citrique ou l'acide phosphorique ou encore au moyen d'un tampon tel que par exemple le phosphate mono et dipotassique et le carbonate acide d'ammonium.

Selon une forme de réalisation préférée, la composition réductrice peut contenir également un agent tensioactif de type non ionique, anionique, cationique ou amphotère.

La composition réductrice peut également contenir des agents traitants, des substances actives tels que l'acide panthoténique, des agents antichutes, des agents antipelliculaires, des épaississants, des agents de suspension, des agents séquestrants, des agents opacifiants, des colorants, des filtres solaires ainsi que des parfums et conservateurs et éventuellement d'autres agents réducteurs.

La composition oxydante est du type couramment utilisée et contient comme agent oxydant de l'eau oxygénée, un bromate alcalin, un persel, un polythionate ou un mélange de bromate alcalin et de persel. La concentration en eau oxygénée peut varier de 1 à 20 volumes et de préférence de 1 à 10, la concentration en bromate alcalin de 2 à 12 % et celle en persel de 0,1 à 15 % en poids par rapport au poids total de la composition oxydante. Le pH de la composition oxydante est généralement compris entre 2 et 8 mais de préférence entre 3 et 6.

On va maintenant donner à titre d' illustration plusieurs exemples du procédé de déformation permanente des cheveux selon l'invention.

### EXEMPLES

### EXEMPLE 1 :

Sur des cheveux mouillés, préalablement enroulés sur des bigoudis d'un diamètre de 14mm, on applique la composition réductrice (A) suivante :

### Composition (A) :

| | |
|---|---|
| - Chlorhydrate de cystéamine | 11,3 g |
| - Chlorure d'oléocétyldiméthylhydroxyéthylammonium en solution aqueuse à 30 % en matière active | 0,39g (Ma) |
| - Ammoniaque qs | pH = 8,5 |
| - Eau qsp | 100 g |

Après avoir laissé agir la composition pendant 15 minutes, on rince les cheveux à l'eau courante et on applique ensuite sur les cheveux la composition (B) suivante :

### Composition (B) :

| | |
|---|---|
| - Formaldéhyde | 0,2 g |
| - Eau qsp | 100 g |

On laisse agir la composition pendant 15 minutes, puis on rince à nouveau à l'eau courante avant d'appliquer la composition oxydante (C) suivante :

### Composition (C) :

| | |
|---|---|
| - Peroxyde d'hydrogène en solution aqueuse à 200 Volumes | 4,8 g |
| - Acide citrique qs | pH = 3 |
| - Eau qsp | 100 |

Après avoir laissé agir la composition oxydante pendant 5 minutes, on rince à l'eau puis on enlève les bigoudis et on sèche les cheveux.

On ne constate pas la formation de mauvaises odeurs dans le temps, même après humidification des cheveux.

A titre de comparaison, le même procédé a été réalisé en omettant le traitement à l'aide de la composition (B) et l'on a constaté qu'après une certaine période de temps, les cheveux dégageaient une odeur désagréable.

### ETUDES COMPARATIVES

### A. Traitement avant l'étape d'oxydation :

Dix mèches de cheveux de 2,5g chacune environ sont traitées à l'aide d'un shampooing puis rincées. On applique ensuite sur chacune des mèches la composition réductrice suivante :

| | |
|---|---|
| Chlorhydrate de cystéamine | 9,6 g |
| Tegobétaïne | 1 g |
| Ammoniaque à 20 % dans l'eau qs | pH 8,5 |
| Eau déminéralisée qs | 100 g |

Après avoir laissé agir pendant 15 minutes, on rince à l'eau l'ensemble des 10 mèches.

Une des mèches est gardée comme témoin et les neuf mèches restantes sont traitées respectivement par les compositions de traitement 1 à 9 suivantes :

### Composition 1

| | |
|---|---|
| 2,4,6-trihydroxybenzaldéhyde | 7,7 g |
| alcool éthylique | 50 g |
| eau déminéralisée qs | 100 g |

### Composition 2

| | |
|---|---|
| α-méthyl-β-(p-tert-butylphényl)propionaldéhyde (vendu sous la dénomination de "Lilial" par la Société GIVAUDAN) | 8 g |
| alcool éthylique qs | 100 g |

### Composition 3

| | |
|---|---|
| 2-méthyl-4-(2,6,6-triméthyl-2(1)-cyclohéxène -1-yl)butanal (vendu sous la dénomination de "Cétonal" par la Société GIVAUDAN) | 9,1 g |
| alcool éthylique qs | 100 g |

### Composition 4

| | |
|---|---|
| 3,4-diméthoxybenzaldéhyde | 6,2 g |
| alcool éthylique qs | 50 g |
| Eau déminéralisée qs | 100 g |

### Composition 5

| | |
|---|---|
| 2,3,4-triméthoxybenzaldéhyde | 9,8 g |
| alcool éthylique qs | 100 g |

### Composition 6

| | |
|---|---|
| 2,5-diméthoxybenzaldéhyde | 8,3 g |
| alcool éthylique qs | 100 g |

### Composition 7

| | |
|---|---|
| 2-héxyl-3-phényl-2-propenal (vendu sous la dénomination de "héxyl cinnamic aldéhyde" par la Société GIVAUDAN) | 10,8 g |
| alcool éthylique qs | 100 g |

### Composition 8

| | |
|---|---|
| Formaldéhyde à 30 % dans l'eau | 3 g |
| Eau déminéralisée qs | 100 g |

### Composition 9

| | |
|---|---|
| 4-hydroxy 3-méthoxybenzaldéhyde | 7,6 g |
| Ammoniaque à 20 % dans l'eau qs | pH 8,5 |
| Eau déminéralisée qs | 100 g |

Après avoir laissé, agir pendant 15 minutes environ, on rince abondamment et on applique sur les neuf mèches ainsi traitées et sur la mèche témoin une composition oxydante constituée de :

| | |
|---|---|
| Eau oxygénée à 8 volumes | 100 g |
| Acide citrique qs | pH3 |

Après un temps de pose de 5 minutes, les dix mèches sont rincées à l'eau.

Après séchage, les mèches sont suspendues dans des compartiments séparés pendant 4 jours.

Après le deuxième jour, les mèches sont soumises à un shampooing, lavées à l'eau et séchées.

Le quatrième jour, les mèches sont vaporisées à l'eau et soumises à un panel de 4 personnes, en vue de déterminer si elles dégagent des odeurs résiduelles. L'intensité de l'odeur est notée selon le barème suivant :
- 0: pas d'odeur
- 1: très faible odeur
- 2: faible odeur
- 3: odeur perceptible
- 4: odeur prononcée
- 5: forte odeur
- 6: très forte odeur

Les résultats obtenus sont rassemblés dans le tableau suivant :

| Mèches N° | Composition de traitement | Note moyenne des odeurs perçues |
|---|---|---|
| 1 (Témoin) | - | 5,75 |
| 2 | 1 | 1 |
| 3 | 2 | 1 |
| 4 | 3 | 1,5 |
| 5 | 4 | 1 |
| 6 | 5 | 1 |
| 7 | 6 | 3 |
| 8 | 7 | 1,25 |
| 9 | 8 | 2,25 |
| 10 | 9 | 2 |

Comme on peut le constater, le traitement des mèches entre l'étape de réduction et l'étape d'oxydation, à l'aide d'une composition contenant un composé mono-aldéhydique, diminue très fortement la présence d'odeurs résiduelles. Seule la composition (6) contenant du 2,5-diméthoxybenzaldéhyde donne un résultat sensiblement inférieur aux autres compositions.

### B. Traitement après l'étape d'oxydation

Selon le même mode opératoire que décrit ci-dessus en (A), on a traité neuf mèches à l'aide de la composition réductrice suivante :

| | |
|---|---|
| Chlorhydrate de cystéamine | 10 g |
| Thioglycolate de glycérol | 3,1 g |
| Tégobétaïne | 1 g |
| Ammoniaque à 20 % dans l'eau qs | pH 7,5 |
| Eau déminéralisée qs | 100 g |

Après avoir laissé poser 15 minutes, on rince à l'eau les mèches et on applique la composition oxydante suivante :

| | |
|---|---|
| Eau oxygénée à 8 volumes | 100 g |
| Acide citrique qs | pH 3 |

On laisse agir 5 minutes puis on traite 8 des mèches (l'une étant gardée comme témoin) par respectivement les conpositions de traitement 10 à 17 suivantes :

### Composition 10

| | |
|---|---|
| 2,4,6-trihydroxybenzaldéhyde | 6 g |
| alcool éthylique | 50 g |
| Eau déminéralisée qs | 100 g |

### Composition 11

| | |
|---|---|
| α-méthyl-β-(p-tert-butylphényl)propionaldéhyde | 5 g |
| alcool éthylique qs | 100g |

### Composition 12

| | |
|---|---|
| 2-méthyl-4-(2,6,6-triméthyl-2(1)-cyclohexene-1-yl) butanal | 5,2g |
| alcool éthylique qs | 100 g |

### Composition 13

| | |
|---|---|
| 3,4-diméthoxybenzaldéhyde | 4,2g |
| alcool éthylique qs | 50 g |
| Eau déminéralisée qs | 100 g |

### Composition 14

| | |
|---|---|
| 2,3,4-triméthoxybenzaldéhyde | 4,85g |
| alcool éthylique qs | 100g |

### Composition 15

| | |
|---|---|
| 2,5-diméthoxybenzaldéhyde | 5,8 g |
| alcool éthylique qs | 100 g |

### Composition 16

| | |
|---|---|
| 2-héxyl-3-phényl-2-propénal | 8,8 g |
| alcool éthylique qs | 100 g |

### Composition 17

| | |
|---|---|
| 4-hydroxy 3-méthoxybenzaldéhyde | 6,8g |
| Ammoniaque à 20 % dans l'eau qs | pH 8,5 |
| Eau déminéralisée qs | 100 g |

Les mèches y compris la mèche témoin sont après 4 jours soumises a un panel de 4 personnes en vue de déterminer si elles dégagent des odeurs résiduelles selon le même barême que celui donné ci-dessus en (A).

Les résultats obtenus sont rassemblés dans le tableau suivant :

| Mèches N° | Composition de traitement | Note moyenne des odeurs perçues |
|---|---|---|
| 1 (Témoin) | - | 5,75 |
| 2 | 10 | 2,25 |
| 3 | 11 | 2 |
| 4 | 12 | 2 |
| 5 | 13 | 1,75 |
| 6 | 14 | 2 |
| 7 | 15 | 3 |
| 8 | 16 | 2,25 |
| 9 | 17 | 1,75 |

Les résultats obtenus montrent que par rapport au témoin, la diminution de l'odeur résiduelle est tout à fait significative. En comparant les résultats des études du traitement, avant ou après l'étape d'oxydation, on peut constater que de façon surprenante, une diminution plus sensible des odeurs résiduelles est obtenue lorsque la composition contenant le composé mono-aldéhydique est appliquée avant la composition oxydante.

## Revendications

1. Procédé de déformation permanente des cheveux comprenant une étape de réduction à l'aide d'une composition à base de cystéamine ou de l'un de ses sels et une étape d'oxydation, caractérisé par le fait qu'avant ou après l'étape d'oxydation, on applique sur les cheveux une composition contenant dans un véhicule cosmétiquement acceptable un composé mono-aldéhydique.

2. Procédé, selon la revendication 1, caractérisé par le fait que le composé mono-aldéhydique est le formaldéhyde ou un aldéhyde aromatique choisi parmi :
le 2,4,6-trihydroxybenzaldéhyde, l'α-méthyl β-(p-tert-butyl phényl) propionaldéhyde, le 2-méthyl-4-(2,6,6-triméthyl-2(1) cyclohéxene-1-yl) butanal, le 2-hexyl-3 phényl 2-propenal, le 3,4-diméthoxybenzaldéhyde, le 2,3,4-triméthoxybenzaldéhyde, le 2,5-diméthoxybenzaldéhyde et le 4-hydroxy 3-méthoxybenzaldéhyde.

3. Procédé selon la revendication 1 ou 2, caractérisé par le fait que le composé mono-aldéhydique est présent à une concentration molaire comprise entre 0,15 et 1,8 mole et de préférence entre 0,22 et 0,8 mole.

4. Procédé selon l'une quelconque des revendications précédentes, caractérisé par le fait que le véhicule cosmétiquement acceptable est une solution aqueuse ou une solution alcoolique ou hydroalcoolique d'un alcool aliphatique inférieur.

5. Procédé selon l'une quelconque des revendications précédentes, caractérisé par le fait que la composition contenant le composé mono-aldéhydique contient en outre au moins un ingrédient cosmétique choisi parmi un agent tensioactif anionique, nonionique ou amphotère ou zwitterionique ou cationique, un agent alcalinisant ou acidifiant, un agent conservateur, un stabilisant, un agent de traitement, un colorant, un filtre solaire, un agent épaississant ou un agent nacrant.

6. Procédé selon l'une des revendications 1 à 5, caractérisé par le fait que les cheveux sont rincés à l'eau préalablement à l'application de la composition contenant le composé mono-aldéhydique.

7. Procédé selon l'une quelconque des revendications précédentes, caractérisé par le fait que la composition contenant le composé mono-aldéhydique est appliquée sur les cheveux pendant un temps de 1 à 60 minutes.

8. Procédé selon l'une quelconque des revendications précédentes, caractérisé par le fait que l'on applique sur les cheveux une composition aqueuse de formaldéhyde et que l'on effectue un rinçage des cheveux à l'eau.

9. Procédé selon l'une quelconque des revendications précédentes, caractérisé par le fait que la composition contenant le composé mono-aldéhydique est appliquée sur les cheveux avant l'étape d'oxydation, les cheveux ayant été préalablement rincés à l'eau après l'étape de réduction.

## Claims

1. Process for the permanent deformation of hair comprising a reduction stage using a composition based on cysteamine or on one of its salts and an oxidation stage, characterized in that, before or after the oxidation stage, a composition containing, in a cosmetically acceptable vehicle, a monoaldehyde compound is applied to the hair.

2. Process according to Claim 1, characterized in that the monoaldehyde compound is formaldehyde or an aromatic aldehyde chosen from:
2,4,6-trihydroxybenzaldehyde, α-methyl-β-(p-tert-butylphenyl)propionaldehyde, 2-methyl-4-(2,6,6-trimethyl-2(1)-cyclohexen-1-yl)butanal, 2-hexyl-3-phenyl-2-propenal, 3,4-dimethoxybenzaldehyde, 2,3,4-trimethoxybenzaldehyde, 2,5-dimethoxybenzaldehyde and 4-hydroxy-3-methoxybenzaldehyde.

3. Process according to Claim 1 or 2, characterized in that the monoaldehyde compound is present at a molar concentration of between 0.15 and 1.8 mol and preferably between 0.22 and 0.8 mol.

4. Process according to any one of the preceding claims, characterized in that the cosmetically acceptable vehicle is an aqueous solution or an alcoholic or aqueous/alcoholic solution of a lower aliphatic alcohol.

5. Process according to any one of the preceding claims, characterized in that the composition containing the monoaldehyde compound additionally contains at least one cosmetic ingredient chosen from an anionic, non-ionic or amphoteric or zwitterionic or cationic surface-active agent, a basifying or acidifying agent, a preserving agent, a stabilizer, a treatment agent, a dye, a sun-screening agent, a thickening agent or a pearlessence agent.

6. Process according to one of Claims 1 to 5, characterized in that the hair is rinsed with water prior to the application of the composition containing the monoaldehyde compound.

7. Process according to any one of the preceding claims, characterized in that the composition containing the monoaldehyde compound is applied to the hair for a time of 1 to 60 minutes.

8. Process according to any one of the preceding claims, characterized in that an aqueous formaldehyde composition is applied to the hair and in that the hair is rinsed with water.

9. Process according to any one of the preceding claims, characterized in that the composition containing the monoaldehyde compound is applied to the hair before the oxidation stage, the hair having been rinsed beforehand with water after the reduction stage.

## Patentansprüche

1. Verfahren zur dauerhaften Verformung Von Haaren, umfassend einen Reduktionsschritt mittels einer Zusammensetzung auf Grundlage von Cysteamin oder eines seiner Salze sowie einen Oxidationsschritt, dadurch gekennzeichnet, daß man vor oder nach dem Oxidationsschritt eine Zusammensetzung auf die Haare aufbringt, die in einem kosmetisch geeigneten Träger eine Monoaldehydverbindung enthält.

2. Verfahren gemäß Anspruch 1, dadurch gekennzeichnet, daß die Monoaldehydverbindung Formaldehyd oder ein aus 2,4,6-Trihydroxybenzaldehyd, l'α-Methyl-β-(p-tert.-butylphenyl)-propionaldehyd, 2-Methyl-4-(2,6,6-trimethyl-2(1)cyclohexen-1-yl)-butanal, 2-Hexyl-3-pheny-2-propenal, 3,4-Dimethoxybenzaldehyd, 2,3,4-Trimethoxybenzaldehyd, 2,5-Dimethoxybenzaldehyd und 4-Hydroxy-3-methoxybenzaldehyd ausgewähltes aromatisches Aldehyd ist.

3. Verfahren gemäß Anspruch 1 oder 2, dadurch gekennzeichnet, daß die Monoaldehydverbindung in einer molaren Konzentration von 0,15 bis 1,8 Mol und vorzugsweise zwischen 0,22 und 0,8 Mol vorliegt.

4. Verfahren gemäß einem der vorstehenden Ansprüche, dadurch gekennzeichnet, daß der kosmetisch geeignete Träger eine wäßrige Lösung oder eine alkoholische oder hydroalkoholische Lösung eines niedrigen aliphatischen Alkohols ist.

5. Verfahren gemäß einem der vorstehenden Ansprüche, dadurch gekennzeichnet, daß die die Monoaldehydverbindung enthaltende Zusammensetzung zusätzlich mindestens einen kosmetischen Bestandteil enthält, der aus einem anionischen, nichtionischen oder amphoteren oder zwitterionischen oder auch kationischen oberflächenaktiven Mittel, einem alkalinisierenden oder acidifizierenden Mittel, einem Konservierungsmittel, einem Stabilisator, einem Pflegemittel, einem Farbstoff, einem Sonnenfilter, einem Dickungsmittel oder einem Perlmuttglanz verleihenden Mittel ausgewählt ist.

6. Verfahren gemäß einem der Ansprüche 1 bis 5, dadurch gekennzeichnet, daß die Haare vor dem Aufbringen der die Monoaldehydverbindung enthaltenden Zusammensetzung mit Wasser gespült werden.

7. Verfahren gemäß einem der vorstehenden Ansprüche, dadurch gekennzeichnet, daß die die Monoaldehydverbindung enthaltende Zusammensetzung für eine Zeitspanne von 1 bis 60 Minuten auf die Haare aufgetragen wird.

8. Verfahren gemäß einem der vorstehenden Ansprüche, dadurch gekennzeichnet, daß man auf die Haare eine wäßrige Formaldehydzubereitung aufbringt und die Haare mit Wasser spült.

9. Verfahren gemäß einem der vorstehenden Ansprüche, dadurch gekennzeichnet, daß die die Monoaldehydverbindung enthaltende Zusammensetzung vor dem Oxidationsschritt auf die Haare aufgetragen wird, wobei die Haare zuvor nach dem Reduktionsschritt mit Wasser gespült wurden.
